# EUROPEAN PATENT APPLICATION

(11) **EP 1 738 750 A1**
(43) Date of publication of application: **03.01.2007**
(21) Application number: 06253325.2
(22) Date of filing: 27.06.2006
(51) Int. Cl.: A61K 9/16, A61K 9/20, A61K 33/10

(54) **Method of granulating calcium carbonate and products provided therefrom**

(30) Priority: 29.06.2005 US 169540
(71) Applicant: J.M. HUBER CORPORATION, Edison, NJ 08837-2220 (US)
(72) Inventor: Liu, Sung-Tsuen, Edison, NJ 08837-2220 (US); Skinner, William, Edison, NJ 08837-2220 (US)
(74) Representative: Jacob, Reuben Ellis

(57) **Abstract**

A method of granulating calcium carbonate is provided. Such a method entails the inclusion of very low amounts of an aqueous solution citric acid to a calcium carbonate material, with subsequent, mixing, drying, milling, and sieving to provide the correct target particle size range for the resultant granulated materials. Such granulated calcium carbonate materials exhibit very high available calcium levels, excellent flow characteristics, capability for compression into tablets, and a drastic reduction in needed binders to effectuate the desired granulation itself. Products including such granulated calcium carbonate materials are also encompassed within this invention.

## Description

The present invention relates generally to a method of granulating calcium carbonate. Such a method entails the inclusion of very low amounts of an aqueous solution citric acid to a calcium carbonate material, with subsequent, mixing, drying, milling, and sieving to provide the correct target particle size range for the resultant granulated materials. Such granulated calcium carbonate materials exhibit very high available calcium levels, excellent flow characteristics, capability for compression into tablets, and a drastic reduction in needed binders to effectuate the desired granulation itself. Products including such granulated calcium carbonate materials are also encompassed within this invention.

Calcium carbonate has been utilized for many years as orally ingested supplements for purposes ranging from antacids to osteoporosis medications. It has been a combined aim for such materials to deliver the highest level of available calcium within a form that permits proper transfer, tablet production, and ultimate ingestion by a target patient. As such, granulation of powdered calcium carbonate (whether in ground or precipitated state) has been a requirement within the subject industries. Without granulation, the calcium carbonate materials would exhibit, as a powder, poor flowability characteristics, cementation during storage and high levels of dusting when transferred, at least, while incorporating such materials into proper orally ingested tablets and/or capsules.

Granulation has typically included the utilization of binder systems to facilitate the massing of calcium carbonate powders into larger particle size granules. Such a method, although well known, and extensively followed, exhibits certain drawbacks that leave room for improvement. For instance, such binders, including, without limitation, starch, gelatin, hydroxypropyl methylcellulose, polyvinylpyrrolidone, and the like, are relatively expensive and/or require relatively high levels of use to effectively provide the needed degree of granulation to occur. In fact, a level of 5.0 parts per hundred parts of calcium carbonate is generally the low level of binder additive present within final granulated calcium carbonate formulations. This produces a material that exhibits at most 95% bioavailable calcium carbonate for utilization by the target patient/user within a typical tablet/capsule, or requires the production of relatively large size tablets and/or capsules to increase the amount of bioavailable calcium carbonate present therein. An increase in the amount of bioavailable calcium carbonate would thus be a desired result in order to reduce binder costs, reduce the amount of additives needed to permit granulation, and reduce pill sizes without restricting or reducing the amount of calcium available to the patient/user during ingestion. All of these benefits would necessarily result without any concomitant loss in flowability of the granulated calcium carbonate in comparison with the typical binder-granulated types as well. To date, although such granulated calcium carbonate is well known and used widely, particularly within the nutrition and pharmaceutical industries, as noted above, such a desirable improvement has not been achieved.

It has now been determined that the highly desired, but previously unavailable, improvements in granulated calcium carbonate materials as noted above may be obtained through a method of granulation without any binders present, through the addition of very small amounts of aqueous citric acid to a calcium carbonate powder. Such a method, described in greater detail below, thus provides the advantage of reducing, or, more preferably, omitting, any presence or utilization of binder additives to the calcium carbonate powder prior to, during, and/or after granulation, thereby providing a granulated material with lower amounts of additive, and resultant very high amounts of bioavailable calcium carbonate (and thus, calcium). Another distinct advantage of this method is the production of a granulated calcium carbonate material that exhibits excellent flow properties such that the transfer and handling of such materials are easily accomplished with simultaneous low dusting results. Such free flow properties also result in the ability to easily dispense the granulates uniformly into capsules with little to no dusting and/or lack of control (and thus possible waste of material) during filling of individual capsules and uniformly feed into an automatic tablet press. Furthermore, another advantage of this method, and thus the resultant calcium carbonate granulates made therefrom, is the compressibility of the granulates into tablets for ingestion by a patient/user as well.

Accordingly, this inventive wet granulation method entails the steps of a) providing a calcium carbonate powder material; b) introducing an aqueous citric acid solution within said powder material of step a) in an amount in which the citric acid present therein is from 0.1 to 5.0 parts per 100 parts (i.e., 0.1-5.0% by weight) of the calcium carbonate material; and c) allowing the citric acid and calcium carbonate to react during mixing to form a resultant granulated calcium carbonate.

Such a novel method permits production of a granulated calcium carbonate that is substantially free of typical granulation binders, exhibits excellent flow characteristics to facilitate transport and handling, and can be compressed into tablets.

For the purposes of this description, the term "calcium carbonate powder" and any variations thereof, is intended to encompass any powdered material of calcium carbonate, including ground calcium carbonate and precipitated calcium carbonate.

The term "aqueous citric acid" is intended to encompass a solution of citric acid in liquid form with any amount of water present. As noted above, however, the concentration of the citric acid within such a solution should be adjusted accordingly when added to the calcium carbonate powder such that the amount of citric acid actually reacted with the powder is from 0.1 to 5 parts per hundred parts of the calcium carbonate.

The inventive method is relatively simple to follow, which is yet another advantage. Basically, a starting calcium carbonate powder is obtained initially. To this powder is added the aqueous citric acid in the concentrations as noted above. Such introduction of citric acid may be performed through any means, including, without limitation, drop-wise addition while stirring, if in smaller batch sizes, or in repetitive streams of the liquid solution at set intervals and in set volumes for each repeated introduction. The rate of addition should be performed in a range of anywhere between 0.1 to 100 ml/min per 350 g of calcium carbonate (or, between 0.000285 to .285 ml/min per gram of calcium carbonate), preferably slower, of from 1 to 25 ml/min per 350 g of calcium carbonate. This repetitive introduction step is potentially preferred as it permits reaction of the citric acid in discrete areas within the provided calcium carbonate powder to best ensure, while stirring, that substantially uniform reaction and resultant granulation occurs. Spraying of the citric acid solution on the powder while or with subsequent stirring is also possible. Generally, it has been found that introduction of the aqueous citric acid quickly may result in lack of proper granulation. As uniformity (or at least substantial uniformity) in granulated materials is the aim in this method, the slow addition while stirring is preferred. The granulation may be performed in any apparatus known in the industry such as mixers having low shear or high shear, fluid bed technology, and the like.

The amount of citric acid to be added to the provided calcium carbonate powder may be of the range of 0.1 to 5 parts per hundred parts of the calcium carbonate, with 0.5 to 5 parts preferred, and even more specific preferred amounts noted within the examples, below. Specific volumes to be added are not critical as the important feature is to properly introduce the citric acid at intervals or through a steady, slow stream while the powder is properly stirred. The concentration of the citric acid in solution will determine the amount of such a reactant to be introduced to the calcium carbonate powder, in essence.

Subsequent to reaction of the two components, the wet mixture is then collected and dried. This drying step may be performed within any well known apparatus, including, without limitation, a spray dryer, a rotary dryer, an oven, a fluid bed drier, and the like. The purpose for drying is to remove the excess water through evaporation to leave a granulated solid calcium carbonate in particulate form. Such a dried particulate may then be milled, again with any standard well known apparatus, including, without limitation, a hammer mill, a ball mill, an air mill, a bead mill and the like. The milled particulate can then either be separated through a sieve to provide narrow ranges of particle size materials, or coupled with any fines separated through sieving. Such sieving thus may be utilized as either a particle size sequestration means, or to ensure the granulates are reduced to their smallest particle sizes. It is then possible, if so desired, to react more citric acid with the remaining fines, or with the fines coupled with the larger particle size materials, in order to further granulate the materials to reduce any waste thereof.

The closest prior art to such a novel method is taught within U.S. Pat. No. 5,759,575 to Gergely et al. Such a disclosure encompasses the production of an effervescent tablet including granulated materials comprising citric acid present in an amount in excess of calcium carbonate, far different from the very low amounts within the present inventive method. Other granulated calcium carbonate teachings include the presence of salts of edible acids, not edible acids alone. Such teachings concern the prevention of premature acid-base reactions of the calcium carbonate and edible acid by requiring the salt of such acids.

Once produced, the desired particles can then be introduced into desired end use formulations and/or forms. For instance, the resultant granulated calcium carbonate materials may be used as produced and introduced into gelatin capsules to provide a calcium supplement for a patient/user. Such a supplement may be utilized as an antacid or as a delivery system for calcium (for various reasons and/or purposes, such as a manner of treating osteoporosis, as one non-limiting example). Tablets or lozenges may also be produced from such materials through compression techniques as well. Such tablets may utilize solely the inventive granulated calcium carbonate materials, or may include certain binders or other additives that act as compression aids to improve the friability of such a tablet formulation. Such binders or compression aids may include, without limitation, gum acacia, maltodextrin, alginic acid, gelatin, guar gum, povidone, pregelatinized starch, glucose, ethylcellulose, carboxymethyl cellulose, microcrystalline cellulose, and hydroxypropyl methylcellulose.

Other additives may be present within either a tablet or capsule form including the inventive calcium carbonate materials depending on the nature of the end use selected. Thus, pharmaceutical actives may be present, including any number of analgesics, acid scavengers, cold remedies and the like. Additional dietary supplement ingredients may be present such as essential minerals (potassium, magnesium, selenium, iron, and the like), vitamins, folic acid, niacin and the like. Excipients may be added to tablets to aid in quick tablet disintegration when placed in the buccal cavity as well. Such excipients include, without limitation, crospovidone, MCC, sodium starch glycolate and calcium silicate, such as RXCIPIENT ® FM1000 from J.M. Huber Corporation. Other additives possible within such formulations include coatings (such as cellulose ethers, gums, and the like) over the tablet or lozenge surface, sweeteners, diluents, flavoring agents, colorants, preservatives, other antacid compounds (such as aluminum hydroxide, magnesium hydroxide, magnesium carbonate, and the like), and other typical additives for such orally administered calcium carbonate tablet compositions.

### EXAMPLES 1-7

The calcium carbonate granules were made by adding 350 g of ground calcium carbonate (GCC), HuberCal® 250 available from J.M. Huber Corporation, Quincy, Illinois, to a Hobart mixing tank. To the GCC, a citric acid solution as binder, prepared by dissolving a specified amount of anhydrous citric acid in 40 g of water was added either manually or utilizing a Masterflex flow meter with microprocessor pump drive at a pumping rate of 10 ml/min. The manual addition used in Example 1 was a drop by drop addition and was completed within about 20 to 25 minutes. For Examples 6 and 7, an additional binder, 3.5 g maltodextrin for Example 6 and 3.5 g Acacia gum for Example 7, was mixed with the citric acid to provide a treatment level of 1% citric acid and 1% additional binder. During the addition of citric acid solution, the Hobart mixer was stirred at medium speed. After all the binder was added, the formed granules were oven dried overnight at 100 °C. The granules were put through a 20 mesh (850µm) U.S. sieve and the granules which did not pass through 20 mesh sieve were gently milled using a mortar and pestle and combined with the granules which previously passed through the 20 mesh sieve. The variables used to make Examples 1-7 granules are summarized in Table 1.

### COMPARATIVE EXAMPLE 1

For comparison, granules were formed by granulating 350 g HuberCal® 250 GCC with 40 ml water and no other binder by the same method as described above in Examples 1-7. The variables used to make Comparative Example 1 are summarized in Table 1.

**TABLE 1**

| Example No. | % Citric Acid | Additional. Binder | Citric Acid, g | Water g | Addition Rate, ml/min |
|---|---|---|---|---|---|
| 1 | 2 | 0 | 7 | 40 | manual |
| 2 | 2 | 0 | 7 | 40 | 10 |
| 3 | 1 | 0 | 3.5 | 40 | 10 |
| 4 | 0.5 | 0 | 1.75 | 40 | 10 |
| 5 | 5 | 0 | 17.5 | 40 | 10 |
| 6 | 1 | Maltodextrin | 3.5 | 40 | 10 |
| 7 | 1 | Acacia Gum | 3.5 | 40 | 10 |
| Comparative Ex 1 | 0 | 0 | 0 | 40 | 10 |

The particle distribution and flow characteristics of the granules produced above were measured according to the methods described below with the results summarized in Table 2.

The particle size distribution was evaluated by placing the 50 g of granules on a stack of 20 mesh (850µm), 50 mesh (180µm), 100 mesh (150 µm), 140 mesh (105 µm), and 200 mesh (75 µm) U.S. sieves with the sieves having the largest openings at the top, i.e. in the order listed with the 20 mesh sieve on top and 200 mesh sieve at the bottom. The sieves were placed on a Boemer Portable Sieve Shaker, Model RX-24, available from W.S. Tyler, Inc., Mentor, Ohio, and shaken for 5 minutes after which the screens were separated and the granules on each sieve were weighed.

The intrinsic flowability, which is the property of a powder to flow evenly under the action of gravity and other forces, was determined using a FLODEX® tester available from Hanson Research, Chatsworth, California. The FLODEX tester is comprised of a funnel with stopper to hold the test powder, under which is a straight-walled open cylinder and finally one of a series of plates with increasing orifice sizes. The FLODEX tester was assembled with the plate having the smallest orifice size and 50 g of the sample was placed in the stoppered funnel. After 30 seconds the stopper was removed and if the sample flowed through the orifice, the size of the orifice diameter, in mm, was recorded as the FLODEX index. If the sample did not flow through the orifice, the sample was placed back in the funnel and the experiment was repeated with plates of increasing orifice size until the sample flowed through an orifice. The diameter of the smallest orifice needed for flow was recorded as the FLODEX index. This test simulates how materials will flow, i.e. to feed a tableting machine.

**TABLE 2**

| % particles | | | | | | | |
|---|---|---|---|---|---|---|---|
| Example No. | FLODEX Index mm | > 20 mesh | 20-50 mesh | 50-100 mesh | 100-140 mesh | 140-200 mesh | <200 mesh |
| 1 | 7 | 0 | 1.1 | 44.5 | 24.8 | 15.1 | 14.5 |
| 2 | 5 | 0 | 13.5 | 30.2 | 18.6 | 17 | 20.8 |
| 3 | 5 | 0 | 4.5 | 21.2 | 15.9 | 21.5 | 36.9 |
| 4 | 14 | 0 | 0.2 | 26.8 | 10.7 | 21.2 | 41 |
| 5 | 6 | 0.8 | 80.2 | 11 | 2.4 | 2.1 | 3.5 |
| 6 | 4 | 0.4 | 23.2 | 50.5 | 11.1 | 5.6 | 9.3 |
| 7 | 4 | 0.3 | 17 | 34.2 | 19 | 14.4 | 15 |
| Comparative Example 1 | 20 | - | - | - | - | - | - |

A FLODEX Index measurement of 15 mm indicates good flowability as a solid; preferably, this measurement should be even lower, with a 10 mm more preferable, and even lower, such as below 7, most preferable. Such a result indicates that the products made within the inventive method exhibit excellent flow characteristics as a granulate material.

### EXAMPLE 8

Granules made in Example 5 were compressed into tablets and several properties of the formed tablets were evaluated. Tablets were prepared by weighing all formulation ingredients (97.5% GCC and 2.0% croscarmellose sodium) together, except the lubricant magnesium stearate, on a weighing pan. Typically, a tablet formulation was 300g to 500g total weight, in order to prepare multiple tablets for testing. The combined ingredients were passed through a 20 mesh (850µm) sieve to remove any lumps and then the resulting mixture was transferred to a PK-V blender (twin shell dry blender model 014-215-0053, available from Patterson Kelly, East Stroudsburg, PA) and mixed for 5 minutes. The magnesium stearate lubricant (0.5%) was then geometrically diluted with the mixture and then added back to the PK blender and all ingredients mixed together for an additional 2 minutes.

Tablets were formed from the resulting formulation on an 8-station Piccola rotary tablet press available from Riva S.A., Argentina, fitted with 10mm standard concave die punches compacting at 15 kN and 30 kN compression forces. Tablet weight was maintained between 750 mg to 800 mg by adjusting the tablet press. Tablet ejection force was measured by the tableting press.

All tablets were prepared 24 hours before testing hardness, disintegration time and friability. Tablet hardness, expressed in kP, was measured on 5 tablets utilizing a Erweka TBH30 instrument (Milford, CT) and the result reported was an average of 5 measurements.

Tablet disintegration time (DT) was determined according to the USP test for uncoated tablets by placing 3 tablets (each tablet in a separate tube) in an Erweka ZT72 disintegrator (Milford, CT). The tablets were repeatedly immersed in 37°C deionized water at a rate of 30 strokes per minute until the tablets disintegrated, as detected and recorded by the instrument. The reported result was an average of the 3 measurements.

Tablet friability was determined by placing 10 tablets in a Distek, Inc. Friabilator DF-3 (North Brunswick, NJ) set for 100 revolutions. The % friability is calculated from the amount of tablet weight lost (friable) by weighing the tablets before and after rotation.

**TABLE 3**

| | | |
|---|---|---|
| Compression Force, kN | 15 | 30 |
| Tablet weight, mg | 788 | 766 |
| Tablet thickness, mm | 5.74 | 5.28 |
| Ejection Force, N | 310 | 512 |
| Tablet Hardness, Kp | 6.56 | 14.08 |
| % Friability | 1.03 | 0.31 |
| DT, seconds | 30 | 16 |

When this granulation was compressed at 30 kN compaction pressure the tablets demonstrated acceptable performance. Tablet hardness, friability and ejection forces were acceptable for tablets compressed on 10 mm tooling.

Such results indicate the acceptability of the resultant calcium carbonate materials of the inventive method as a source for compressed tablets.

While the invention will be described and disclosed in connection with certain preferred embodiments and practices, it is in no way intended to limit the invention to those specific embodiments, rather it is intended to cover equivalent structures structural equivalents and all alternative embodiments and modifications as may be defined by the scope of the appended claims.

## Claims

1. A method for the wet granulation of calcium carbonate, comprising the steps of:
a) providing a calcium carbonate powder material;
b) introducing an aqueous citric acid solution to said powder material in an amount in which the citric acid present therein is from 0.1 to 5.0 parts per 100 parts of the calcium carbonate material; and
c) allowing the citric acid and calcium carbonate to react during mixing to form a resultant granulated calcium carbonate.

2. The method of claim 1, wherein the introduction of the aqueous citric acid in step b) is accomplished through dropwise addition at a rate of from 0.1 to 100 ml/min per 350 grams of calcium carbonate powder material.

3. A granulated calcium carbonate produced by the method of claim 1 or 2, wherein said granulated material exhibits a FLODEX index measurement of at most 15 mm.

4. The granulated calcium carbonate according to claim 3, wherein said granulated material exhibits a FLODEX index measurement of at most 10 mm.

5. The granulated calcium carbonate according to claim 3, wherein said granulated material exhibits a FLODEX index measurement of at most 7 mm.

6. A tablet or capsule, comprising the granulated calcium carbonate of any of claims 3 to 5.

7. A tablet or capsule according to claim 6, further comprising a pharmaceutically active compound, or a dietary supplement.

8. A tablet or capsule according to claim 7, the pharmaceutically active compound comprising an analgesic, an acid scavenger and/or a cold remedy.
